# EUROPÄISCHE PATENTSCHRIFT

(11) **EP 2 840 978 B1**
(45) Veröffentlichungstag und Bekanntmachung des Hinweises auf die Patenterteilung: **24.08.2022**
(21) Anmeldenummer: 13719531.9
(22) Anmeldetag: 26.04.2013
(51) Int. Cl.: A61B 17/122, A61B 17/08

(54) **EINTEILIGER CHIRURGISCHER CLIP**
ONE-PART SURGICAL CLIP
PINCE CHIRURGICALE EN UNE SEULE PIÈCE

(30) Priorität: 27.04.2012 DE 102012103727
(43) Veröffentlichungstag der Anmeldung: 04.03.2015
(73) Patentinhaber: Aesculap AG, 78532 Tuttlingen (DE)
(72) Erfinder: ZIERIS, Gerold, 78570 Mühlheim (DE)
(74) Vertreter: Winter, Brandl - Partnerschaft mbB
(86) Internationale Anmeldenummer: PCT/EP2013/058738
(87) Internationale Veröffentlichungsnummer: WO 2013/160452

(56) Entgegenhaltungen:
- EP-A1- 0 122 046
- WO-A1-2011/068073
- DE-A1- 2 036 725
- DE-C1- 19 858 580
- US-A- 4 337 774
- US-A- 4 835 824
- US-A- 5 236 440
- US-A1- 2008 077 144

## Beschreibung

Die vorliegende Erfindung betrifft einen chirurgischen Clip (insbesondere einen einteiligen Aneurysmenclip) gemäß dem Oberbegriff des Patentanspruchs 1.

Chirurgische Clips (oder auch Gewebeclip genannt) sind medizinische Implantate, die zumeist temporär zum Verschließen beispielsweise von Gewebeperforationen oder zur Therapie von Aneurysmen als Gefäßklammern eingesetzt werden. Hierfür eignen sich ein Vielzahl von Clipformen beginnend mit maulartigen Clips, bei denen die Klemm- oder Clipbranchen ähnlich zu einem Ober- und Unterkiefer als zwei einander gegenüberliegende, längs gebogene Zahnreihen ausgeformt sind, die an ihren jeweiligen beiden Längsenden über Scharniere miteinander gekoppelt sind, bis hin zu reihenartigen (geraden) Clips, die vergleichbar zu einer herkömmlichen Zange zwei sich gegenüberliegende im Wesentliche gerade Klemmschienen haben, die ggf. mit Zähnen oder einer Riffelung besetzt sind und die nur an jeweils einem Längsende miteinander scharnierartig gekoppelt sind.

Allen bekannten Clipformen jedoch gemein ist zumindest eine Federanordnung, die eine Klemmkraft auf die Clipbranchen anlegt. Diese zumindest eine Federanordnung kann als ein separates Bauteil ausgeführt sein, das in den Clip eingesetzt ist, um die Clipbranchen gegeneinander vorzuspannen oder sie ist in den Clip (einstückig) integriert. Im letzteren Fall bildet die Federanordnung im Wesentlichen das Scharnier bzw. die Scharniere, mit welchem(n) die beiden Clipbranchen schwenkbar gekoppelt sind.

Aus dem Stand der Technik ist ein chirurgischer Clip der Gattung mit geraden Clipbranchen und einseitiger Federanordnung bekannt, wie er beispielsweise in der DE 20 2010 008 512 U1 veröffentlicht ist.

Die Klemm- oder Schließkraft wird hier durch eine Schenkelfeder erzeugt, die entweder als Rund- oder Rechteckfeder ausgebildet ist. Im Konkreten besteht ein solcher Clip aus zwei Clipbranchen, die sich in ihren jeweiligen Mittenabschnitten lose (d.h. ohne mechanische Verbindung wie z.B. ein Scharnierstift) überkreuzen, um zwei in Schließposition parallel aneinanderliegende Klemmabschnitte und zwei in dieser Position voneinander beabstandete Betätigungsschenkel bzw. Betätigungsabschnitte zu bilden. Die freien Enden der Betätigungsabschnitte sind über die vorstehend genannte Schenkelfeder miteinander verbunden, die entweder einstückig (aus einem einzigen Teil) mit den Clipbranchen oder als separates Bauteil ausgeformt und anschließend mit den Clipbranchen zu einem Teil verbunden (verlötet, verschweißt, zusammengesteckt, etc.) ist. Die Schenkelfeder kann hierbei entweder eine halbe, eine 1.5-fache oder sogar eine 2,5-fache Wicklung besitzen.

Zur Betätigung wird der chirurgische Clip an seinen Betätigungsabschnitten zusammengedrückt, wodurch sich die Klemmabschnitte aufgrund der sich überkreuzenden Ausrichtung der Clipbranchen voneinander beabstanden. Gleichzeitig wird die Schenkelfeder stärker vorgespannt. Werden die Betätigungsabschnitte freigegeben, bewirkt die Schenkelfeder ein Auseinanderdrücken der Betätigungsabschnitte, bis die Klemmabschnitte gegeneinander gepresst sind.

Obgleich die vorstehende Konstruktion eine ausreichend hohe Anpresskraft zwischen den beiden Klemmabschnitten gewährleistet und die Schenkelfeder aufgrund eines nur geringen elastischen Deformationsgrads wenig belastet wird und daher sehr haltbar ist, ergeben sich doch einige Nachteile.

So erfordert die Verwendung der Schenkelfeder einen vergleichsweise aufwändigen und schwierigen Herstellungsvorgang für das Wickeln der Feder, das ohne Rissbildung des Federmaterials erfolgen muss. Auch besteht hierbei die Möglichkeit eines Überwickelns der Schenkelfeder, wodurch sich die Anpresskraft zwischen den Klemmabschnitten verringert. Insgesamt bedarf die Herstellung der Schenkelfeder daher ein hohes Maß an Präzision, um Instrumente herzustellen, deren Eigenschaften (Anpresskraft, Haltbarkeit, Betriebssicherheit etc,) innerhalb eines engen Toleranzbereichs liegen. Die Ausschussquote ist daher entsprechend hoch. Sämtliche genannten und weitere Nachteile des bekannten chirurgischen Clips resultieren letztlich in einem vergleichsweise hohen Preis für den chirurgischen Clip.

Weitere chirurgische Clips dieser Gattung sind beispielsweise auch aus der US 6 179 850 B1 oder der EP 0 122 046 A1 bekannt.

Aus einem weiteren Stand der Technik gemäß der DE 20 2010 008 714 U1 ist ein chirurgischer Clip bekannt mit zwei Clipbranchen, die sich parallel zueinander erstrecken ohne sich zu kreuzen und die in ihren jeweiligen Mittenbereichen über eine stegförmige Biegefeder miteinander verbunden sind. Die Clipbranchen sind demzufolge über die Biegefeder in deren Längsrichtung hinaus verlängert und bilden so zwei Klemmabschnitte auf der einen Seite der Biegefeder und zwei Betätigungsschenkel / -abschnitte auf der anderen Seite der Biegefeder, etwa nach dem Prinzip einer allgemein bekannten Wäscheklammer. Werden demnach die beiden Betätigungsabschnitte auf der einen Seite der stegförmigen Biegefeder gegeneinander gedrückt, bewegen sich die beiden Klemmabschnitte auf der anderen Seite der Biegefeder voneinander weg und umgekehrt.

Dieser chirurgische Clip ist aus einem einzigen Teil nach einem Spritzgussverfahren gefertigt und ist damit hinsichtlich seiner Herstellung relativ preisgünstig. Durch das Spritzgussverfahren ist im Gegensatz zum vorstehend beschriebenen Clip die Fertigung im Wesentlichen maschinell durchführbar und damit kostengünstig. Die Vorspannung der Biegefeder in der geschlossenen Position des Clip wird nach dieser Offenbarung durch eine lokal unterschiedliche Materialschwindung bei Erhärtung des Spritzgussmaterials erreicht. Damit kann auf einfache Weise ein vergleichsweise hoher Qualitätsstandart erreicht werden. Aber auch diese Lösung hat Nachteile.

Obgleich die Biegefeder eine offene, in Richtung hin zu den Betätigungsabschnitten ausbauchende Ringform aufweist, ist der Deformationsweg/-grad der Biegefeder im normalen Gebrauch vergleichsweise groß, sodass die Feder schnell ermüden kann. Außerdem ist die Biegespannung gegenüber der Schenkelfeder viel höher, wodurch auch häufiger Federbrüche möglich sind. Dies resultiert letztlich in einer gegenüber der Schenkelfeder geringeren Betriebssicherheit und Betriebsdauer. Insbesondere hat es sich aber auch als schwierig erwiesen, ausreichende reproduzierbare Klemmkräfte durch die vorstehend beschriebene unsymmetrische Materialschwindung zu erreichen.

Des Weiteren hat es im Stand der Technik auch Bestrebungen dahingehend gegeben, die ring- oder U-förmige Biegefeder an den jeweils einen äußeren Enden der Clipbranchen anzuordnen, so dass Klemm- und Betätigungsabschnitte jeder Clipbranche auf ein und derselben Seite der Feder zu liegen kommen und somit die gleiche Bewegungsrichtung bei der Betätigung des Clips aufweisen (und nicht entgegengesetzt wie beim vorstehend genannten Stand der Technik). In diesem Fall erfolgt ein Aufspreizen des Clips durch Auseinanderziehen der Betätigungsabschnitte und nicht durch deren Zusammendrücken, wie im eingangs genannten Stand der Technik. Dies führt dazu, dass der Clip unhandlich / unergonomisch wird, wodurch sich sein Einsatzbereich deutlich verkleinert. Alternativ besteht grundsätzlich die Möglichkeit, die Branchen zu kreuzen, sodass ein Zusammendrücken der Betätigungsabschnitte ein Öffnen der Klemmabschnitte bewirkt. Aber auch in diesem Fall hat sich zumindest die Vorspannkraft der Feder als vergleichsweise gering erwiesen, sodass die Materialstärke der Feder erhöht werden musste. Der Clip erhält daher insgesamt eine so große Abmessung, dass er sich nur für spezielle Einsatzzwecke eignet.

Ein weiterer chirurgischer Clip ist aus der DE 198 58 580 C1 bekannt. Der darin beschriebene Clip ist U-förmig ausgebildet und hat zwei Clipbranchen, die sich parallel zueinander erstrecken ohne sich zu kreuzen und die in ihren jeweiligen Mittenbereichen über eine stegförmige Biegefeder miteinander verbunden sind. Ein Rastmittel des Clips fixiert den Steg beim Zusammendrücken der Clipbranchen, um dadurch vergleichsweise hohe Haltekräfte zu bewirken. Allerdings hat es sich auch hier als schwierig erwiesen, ausreichend reproduzierbare Klemmkräfte zu erzeugen.

Die US 4 337 774 A offenbart einen mico-chirurgischen Clip zum Klammern von kleinen Blutgefäßen mit integral ausgebildeten Armen, die durch einen Verbindungsstab einstellbar gespannt werden.

Die US 5 236 440 A offenbart einen chirurgischen Verbinder mit zwei Verriegelungsarmen, die von einem Formmesser in das Gewebe gedrückt werden und dann in einer Verriegelungsausnehmung an einem horizontalen Brückenelement einrasten bzw. in den Arm selbst einrasten.

Die US 2008/077144 A offenbart eine selbst-öffnende Hautklammer. Verformbare Beine weisen einen inneren Abschnitt auf, der mit einem Kragen verbunden ist und einen äußeren Abschnitt, der mit Zähnen ausgebildet ist, an denen der Kragen justiert werden kann. Der verformbare innere Abschnitt kann durch eine Öffnung, die von dem äußeren Abschnitt gebildet wird, gezogen werden und so die Klammer schließen.

Die WO 2011/068073 A1 offenbart einen chirurgischen Clip. Der Clip ermöglicht es, ohne Pinzette angebracht und entfernt zu werden. Der Clip weist einen Klemmabschnitt mit Füßen auf, zum Halten eines Objekts, einen überkreuzenden Bereich und einen Halter, der die Federspannung aufbringt.

Die US 4 835 824 offenbart eine medizinische Klammer aus einem einzigen Plastikstreifen, der zwei Arme der Klammer mittels eines Hakens kurzschließt, womit die Klammer geschlossen wird.

Die DE 20 36 725 A1 offenbart eine Plastikklammer für chirurgische Zwecke. Die Klammer ist aus einem Stück Plastik gepresst mit zwei flexiblen Armen und einem gebogenen flexiblen Teil, der die Arme verbindet. Eine Zahnstange an einem Arm und ein Verschlussteil an dem anderen Arm greifen ineinander, so dass die zwei Arme verriegelt werden.

Die DE 198 58 580 C1 offenbart einen Gefäßclip mit zwei über einen verformbaren Steg miteinander verbundenen Schenkeln, wobei an dem Steg zusammenwirkende Rastmittel angeordnet sind, die den Steg festlegen.

Die EP 0 122 046 A1 offenbart eine absorbierbare Verriegelungseinrichtung mit interner Verriegelungseinrichtung. Eine chirurgische Vorrichtung mit einem paar Beinen, die an ihren proximalen Enden miteinander verbunden sind, werden mittels eines Verriegelungsmechanismus in der Nähe der proximalen Enden verriegelt.

Angesichts dieser Problematik ist es die Aufgabe der vorliegenden Erfindung, einen preiswerten chirurgischen Clip mit verbesserten Eigenschaften bereitzustellen. Ein Ziel ist es hierbei, dass der Clip eine hohe Betriebssicherheit insbesondere bezüglich des Funktionserhalts der Vorspannfeder aufweist und möglichst vielseitig einsetzbar ist. Ein weiteres/anderes Ziel ist es, dass der Clip bei kleinstmöglicher Bauweise eine größtmögliche Schließkraft und Öffnungsweite bietet. Zudem sollte der Clip vorzugsweise einen (integrierten) Schutzmechanismus vor mechanischer Überlastung beispielsweise in Folge von unsachgemäßer Handhabung haben.

Die Aufgabe sowie die weiteren Ziele werden durch einen chirurgischen Clip (einen einteiligen bzw. stoffeinstückigen Aneurysmenclip) mit den Merkmalen des Anspruchs 1 gelöst. Vorteilhafte Ausgestaltungen der Erfindung sind Gegenstand der Unteransprüche.

Ein Grundgedanke der Erfindung besteht prinzipiell darin, vorzugsweise für einen stoffeinstückigen chirurgischen Clip der nicht sich überkreuzenden Bauart eine Biegefeder bzw. Biegefeder - (Reihen) - Anordnung zu verwenden, deren Gesamtfederlänge durch (vorzugsweise faltenartige) serielle Anordnung bzw. Aneinanderreihung von Einzelfedern (Einzelblattfedern) bzw. Federarme beispielsweise durch serielles (stoffeinstückiges) Aneinanderfügen / Ausformen einer Anzahl von Biegefedern (Blattfedern), durch plastisches/bleibendes Falten oder Biegen (plastische Deformation während des Herstellungsprozesses) einer einzigen Biegefeder zur Ausbildung einer Anzahl von singulär wirkenden Federabschnitten oder dergleichen stirnseitigen Biegefederverbindungen ausgeweitet ist. An zumindest einem (Mitten-) Abschnitt der Biegefeder - (Reihen) - Anordnung ist eine Kopplungsvorrichtung angeordnet oder aus/angeformt, mittels der zumindest zwei Einzel - (Biege) - Federn oder Federabschnitte (Federarme) unter elastischem Vorspannen zumindest eines dieser zwei Einzel - (Biege) - Federn oder Federabschnitte (Federarme) sowie ggf. unter Verkürzung der aktiven Gesamtfederlänge unmittelbar verbindbar / koppelbar sind.

Grundsätzlich besteht bei einem stoffeinstückigen chirurgischen Clip das Problem, dass bei dessen Herstellung (in Konstruktionslage) beispielsweise nach dem Spritzgussverfahren die Biegefeder-(Anordnung) fertigungstechnisch zunächst nicht vorgespannt ist. Daher bevorzugen die meisten stoffeinstückigen chirurgischen Clips die sich überkreuzende Bauart, wofür die Biegefeder-(Anordnung) elastisch verformt werden muss, damit die zunächst parallelen, nicht gekreuzten Clipbranchen überkreuzt und nunmehr quasi seitenverkehrt gegeneinander gedrückt werden können.

Bei der vorliegenden Erfindung ist es hingegen prinzipiell vorgesehen, die Biegefeder - (Reihen) - Anordnung nach deren mit den Branchen stoffeinstückigen Fertigung in sich vorzuspannen, indem die Biegefederanordnung in Fertigungslage (bei der stoffeinstückigen Herstellung) quasi "offen" ausgebildet ist und zum "Schließen" anschließend erst gespannt werden muss, wodurch eine ausreichende Federvorspannung auch bei chirurgischen Clips der nicht sich überkreuzenden Bauart erreicht werden kann. D.h. die Kopplungsvorrichtung der Biegefeder - (Reihen) - Anordnung dient dazu, wenigstens zwei Biegefederabschnitte / -arme unter deren elastischer Verformung "kurz zu schließen" und damit eine (erhöhte) Federvorspannung in Clip-Schließrichtung zu erreichen.

In der praktischen Anwendung ist es im Wesentlichen so, dass die die zumindest zwei kurz zu schließenden Biegefederabschnitte / -arme an ihren jeweils einen Enden bei der Clipherstellung über ein Zwischenstück (ist ein Bestandteil der Biegefeder - (Reihen) - Anordnung) miteinander verbunden sind, das beim Kurzschließen der beiden Enden scharnierartig (ggf. auch federelastisch) deformiert wird. Durch diese technisch konstruktive Maßnahme kann grundsätzlich auch der Vorteil erzielt werden, dass die Biegefeder - (Reihen) - Anordnung beispielsweise bei Auseinanderbewegen der über diese Federanordnung gekoppelten Clipbranchen die gesamte (maximale) Federlänge (einschließlich des Zwischenstücks) genutzt werden kann, um einem Überbiegen der Federanordnung vorzubeugen und die zu überwindende Federkraft möglichst klein zu halten. Gleichzeitig kann die maximale Federkraft insbesondere in Schließposition der Clipbranchen gesteigert werden, indem die Kopplungsvorrichtung (beispielsweise mit Erreichen der Schließposition) betätigt und die Biegefeder - (Reihen) - Anordnung dadurch quasi kurzgeschlossen wird, d.h. ein Teil der Biegefeder- (Reihen) - Anordnung (nämlich das Zwischenstück) aus dem relevanten Kraftfluss genommen/geschaltet wird. Auf diese Weise könnten durch die Kopplungsvorrichtung theoretisch unterschiedliche Biegefeder - (Reihen) - Anordnungssysteme mit zueinander unterschiedlichen Federeigenschaften/Steifigkeiten eingestellt (bzw. simuliert) werden.

Entscheidend ist jedoch, dass die nachträgliche Vorspannung der Biegefeder - Anordnung nicht (nur) durch ein sich Überkreuzen der Clipbrachen erreicht werden muss sondern durch elastisches Zusammenführen zumindest zweier Biegefederabschnitte / - arme über die erfindungsgemäße Kopplungsvorrichtung erfolgt.

Ein Grundgedanke der Erfindung sieht vor, dass sich die Biegefeder oder die Biegefeder - (Reihen) - Anordnung zumindest teilbereichsweise ausgehend von den Betätigungsabschnitten der Clipbranchen nach innen, d.h. in Richtung hin zu den Klemmabschnitten erstreckt. Auf diese Weise kann der Biegegrad der Biegefeder/Biegefeder - (Reihen) - Anordnung bzw. der Einzelfedern reduziert und damit die Betriebssicherheit erhöht werden. Auch ist aufgrund der Verwendung von Biegefedern eine ausreichend hohe Vorspannkraft erzielbar, ohne dass der chirurgische Clip baulich vergrößert/verlängert werden muss.

Vorzugsweise ist die Kopplungsvorrichtung so gestaltet, dass für deren Kopplungsschluss (Einrückzustand) zumindest die eine Einzelfeder bzw. der eine Federabschnitt elastisch verformt werden muss (vorzugsweise beide zu koppelnden Federabschnitte / - arme), wodurch die zuvor genannte Vorspannung der Biegefeder - (Reihen) - Anordnung in Schließposition der Clipbranchen erreicht wird, deren Höhe von der Dimensionierung der Kopplungsvorrichtung und/oder deren Position längs der Biegefeder - (Reihen) - Anordnung abhängig ist. So kann die Vorspannkraft in Schließposition der Clipbranchen durch Einrücken der Kopplungsvorrichtung in vorbestimmter Weise sicher erreicht / gesteigert werden.

Der chirurgische Clip gemäß einem besonderen Aspekt der Erfindung hat folglich zwei (in Konstruktionslage sich nicht überkreuzende) Clipbranchen jeweils zumindest bestehend aus einem Klemmabschnitt und einem Betätigungsabschnitt, sowie die Biegefeder - (Reihen) - Anordnung, über welche die beiden Clipbranchen, insbesondere deren Betätigungsabschnitte (an deren proximalen Enden) stoffeinstückig miteinander gekoppelt sind. Die Biegefeder - (Reihen) - Anordnung bildet zumindest funktional und/oder konstruktiv mindestens zwei Federschenkel oder Arme, die seriell (über das Zwischenstück) miteinander verbunden sind. Diese zumindest zwei Federarme sind ferner so am Clip ausgerichtet/angeordnet, dass sie sich zwischen den beiden gegenüberliegenden Betätigungsabschnitten in Richtung hin zu den Klemmabschnitten erstrecken.

Durch diese technische Maßnahme wird die maximal mögliche Biegestrecke der Feder (d.h. die maximal wirksame Gesamtfederarmlänge) vergrößert und somit deren Verformungsgrad bzw. der Verformungsgrad der einzelnen Federarme (bei geöffneter / ausgerückter Kopplungsvorrichtung) verkleinert. Die Gesamtabmessung des Clip wird jedoch nicht oder nur unwesentlich vergrößert (verlängert), da sich die Einzelfedern bzw. Federabschnitte/Arme nicht in Richtung weg (in Richtung proximal) von den Klemmabschnitten erstrecken, wie dies im Stand der Technik der Fall ist, sondern in die entgegengesetzte Richtung (in Richtung distal). Auf diese Weise kann eine hohe Vorspannkraft bei geringer Bruchgefahr und kompakter Bauweise erreicht werden, sodass auch der Einsatzbereich des Clip aufgrund dieser kompakten Bauweise erweitert wird. Da der Clip zudem eine Biegefeder (im Wesentlichen zweidimensional) und keine Spiral- oder Schenkelfeder mit komplizierter (dreidimensionaler) Geometrie verwendet, kann dessen Herstellung einfach und damit preisgünstig erfolgen.

Vorteilhaft ist es, wenn die funktional und/oder konstruktiv zumindest zwei seriell angeordneten Federarme der Biegefeder bzw. Biegefederanordnung (in ihrer gemeinsamen Draufsicht) einen C-, U- oder V-förmigen Grundriss bilden, wobei die bügelförmigen Betätigungsabschnitte der Draufsichtform der Federarme angepasst sind. D.h. die Betätigungsabschnitte erstrecken sich einstückig mit den jeweils zugeordneten Klemmabschnitten von deren jeweiliger Wurzel aus in Richtung proximal vorzugsweise in einer C- oder Bogenform, wobei der jeweils eine Federarm am proximalen Ende des entsprechenden Betätigungsabschnitts/-bügels an diesen stoffeinstückig angeformt ist und sich in die entgegen gesetzte Richtung (in Richtung distal) längs des jeweiligen Betätigungsabschnitts (unter Beibehaltung eines im Wesentlichen gleichmäßigen Zwischenspalts) erstreckt.

Eine solche einfache geometrische Grundform ist leicht und präzise herzustellen und trägt somit weiter zur Kostenreduktion bei. Dabei sei an dieser Stelle darauf hingewiesen, dass auch andere Formen für die Federarme (und die Betätigungsabschnitte) wie eine π-oder Ω-Form möglich sind oder die vorstehenden Grundformen durch weitere Unterformen wie in sich gewellte oder in Zick-Zack-Form ausgebildete Federarme überlagerbar sind, wodurch sich die wirksame Federlänge pro Federarm noch weiter vergrößern lässt. Des Weiteren wird durch den konstruktiven Umstand, dass die Biegefeder/Biegefederanordnung des chirurgischen Clip über ihre Federschenkel / -arme einstückig mit den axialen freien (proximalen) Enden der Betätigungsabschnitte der beiden Branchen verbunden ist, eine besonders günstige Biegekrafteinleitung in die Biegefeder (ohne zusätzliche Hebelwirkung) erreicht.

Hier sei darauf hingewiesen, dass der Betätigungsabschnitt jeder Clipbranche starr oder zumindest über eine Teilstrecke (oder vollständig) federelastisch sein kann und somit einen Bestandteil der Biegefederanordnung bilden kann. In anderen Worten ausgedrückt besteht die Möglichkeit, die Biegefeder / Biegefederanordnung funktional/konstruktiv mit zwei Federarmen auszustatten (wie vorstehend beschrieben), derart, dass sich die freien Enden der Federarme in eine Richtung weg von den Klemmabschnitten der Clipbranchen erstrecken. In diesem Fall sind die freien Enden dieser zwei Federarme mit den hinteren Endbereichen/Enden der Betätigungsabschnitte verbunden. Werden nunmehr die Betätigungsabschnitte manuell zusammengedrückt, erfahren diese zwei Federarme eine elastische Biegeverformung längs ihrer Armlänge, d.h. die (Spitz-) Kehre wird elastisch aufgespreizt. Der Verformungsgrad der Biegefederanordnung ergibt sich demnach ausschließlich aus der Gesamtlänge dieser beiden Federarme für den Fall von vergleichsweise starren Betätigungsabschnitten.

Es besteht aber auch die Möglichkeit, die Betätigungsabschnitte zumindest über eine Teillänge oder vollständig elastisch zu gestalten, d.h. bewusst einer elastischen Verformung im normalen Betätigungsfall zuzuordnen. In diesem Fall ergibt sich eine Biegefeder bzw. eine Biegefederanordnung mit den beiden vorstehend genannten Federarmen, an die sich jeweils endseitig ein weiterer Biegefederarm seriell anschließt, sodass im Grundriss eine W-förmige Biegefederanordnung (mit 4 Einzelfedern) gebildet wird. Die Ausrichtung der Biegefederanordnung bleibt hierbei unverändert. D.h. die beiden äußeren Federarme bilden nunmehr gleichzeitig die Betätigungsabschnitte oder zumindest eine Teilstrecke hiervon, wobei deren freie (hinteren) Enden an die zwischen den Betätigungsabschnitten angeordneten Federarmen seriell gekoppelt sind. Auf diese Weise kann der Verformungsgrad jedes einzelnen Federarms weiter reduziert werden.

Ein weiterer ggf. unabhängiger Aspekt der Erfindung betrifft die besondere Ausgestaltung der Kopplungsvorrichtung.

Vorzugsweise ist die Kopplungsvorrichtung in jenem Abschnitt der Biegefeder - (Reihen) - Anordnung angeordnet, in welchem die zumindest zwei Einzelfedern (Federabschnitte) miteinander seriell verbunden sind, d.h. in einem den Klemmbranchen am Nächsten liegenden (Mitten-) Abschnitt. Im Konkreten sind die zumindest zwei, vorzugsweise C-förmigen Einzelfedern oder Federarme weiter vorzugsweise über ein zusätzliches, U- oder V-förmiges Federelement als das Zwischenstück miteinander (stoffeinstückig) seriell verbunden, das sich in die erneut entgegen gesetzte Richtung (in Richtung proximal) erstreckt, d.h. das sich in Richtung hin zu den Klemmbranchen (in Richtung distal) öffnet. Dadurch entstehen vorzugsweise zwei zusätzliche Einzelfedern /Federarme, wodurch die Biegefeder - (Reihen) - Anordnung in Draufsicht nährungsweise die Form eines " ω "-Zeichens erhält. Die Kopplungsvorrichtung ist nunmehr so angeordnet und/oder ausgebildet, dass dieses zusätzliche Federelement kurzgeschlossen, bzw. gebrückt werden kann, sodass in eingerücktem Zustand der Kopplungsvorrichtung nur die beiden äußeren (C-förmigen) Einzelfedern aktiv sind und in ausgerücktem Zustand die beiden inneren (U- oder V-förmig angeordneten) Einzelfedern in den Kraftfluss zugeschaltet sind. Alternativ hierzu ist es aber prinzipiell auch möglich, das vorzugsweise U- oder V-förmige Zwischenstück als Scharnier ohne oder mit nur geringer Federeigenschaft auszugestalten.

In einer vorteilhaften Ausführungsform besteht die Kopplungsvorrichtung aus zwei ineinander verrastbaren Fortsätzen in den Bereichen der Biegefeder - (Reihen)-Anordnung, in welchen das zusätzliche U- oder V-förmige innere Federelement (Zwischenstück) mit den äußeren Einzelfedern (Federarmen) seriell verbunden (angeschlossen) ist. Vorzugsweise ist einer der Fortsätze in Form einer hakenartigen Verlängerung/Rastnase zu der einen Einzelfeder ausgeführt, wohingegen der andere Fortsatz/Rastkante vorliegend in Verlängerung zu einem gegenüberliegenden Schenkel des zusätzlichen U- oder V-förmigen Federelements ausgerichtet ist, sodass die hakenförmige Verlängerung/Rastnase bei Zusammendrücken des U- oder V-förmigen Federelements (Zwischenstücks) den gegenüberliegenden Fortsatz/Rastkante formschlüssig hintergreifen und mit diesem verrasten kann.

Nach einem anderen oder zusätzlichen Aspekt der Erfindung ist der Clip von der geraden Branchenbauart vorzugsweise ein Aneurysmen-Clip, dessen zwei sich gegenüberliegende Klemmabschnitte zumindest bereichsweise jeweils einen gerade sich erstreckenden Balken vorzugsweise mit Zahn- oder Riffelungsbesatz aufweisen.

Ein anderer oder zusätzlicher Aspekt der Erfindung sieht vor, dass am proximalen Ende jedes Betätigungsabschnitts jeweils eine Anschlagsfläche / Anschlagsplatte ausgeformt ist, die bei vollständig geöffnetem (aufgebogenem) Clip, d.h. bei zusammengedrücken Anschlagsplatten aneinander anliegen und somit ein weiteres Öffnen des Clips verhindern. Dies stellt eine konstruktiv einfache Maßnahme gegen ein Überdehnen der Biegefeder und somit einen Schutz gegen Materialermüdung oder Federbruch dar. Darüber hinaus kann der Clip auf einfache Weise in Offenposition gehalten werden, indem die beiden Anschlagsplatten beispielsweise mittels einer Klammer oder Zwinge zusammengehalten werden.

Der erfindungsgemäße chirurgische Clip nach einem anderen oder zusätzlichen Aspekt ist ein Blechbauteil, das vorzugsweise durch Stanzen oder Laserschneiden bearbeitet wird. Außerdem kann der erfindungsgemäße chirurgische Clip auch durch ein Spritzgussverfahren hergestellt werden. Die Biegefeder kann zudem aus einem separaten Federstahl gefertigt und mit den beiden Clipbranchen (stoff-) einstückig vorzugsweise durch Löten oder Schweißen verbunden sein. Alternativ kann der chirurgische Clip einstückig aus einem einzigen Material gefertigt und z.B. abschnittsweise entsprechend seiner Funktion wärmebehandelt sein.

Die Erfindung wird nachstehend anhand eines bevorzugten Ausführungsbeispiels unter Bezugnahme auf die begleitenden Figuren näher erläutert.
- Fig. 1: zeigt einen erfindungsgemäßen chirurgischen Clip (oder Gewebeclip) der geraden Branchenbauart beispielsweise zur Verwendung als Aneurysmenclip in Herstellungslage und damit spannungsfrei (bei geöffneter Kopplungsvorrichtung),
- Fig. 2: zeigt in Vergrößerter Darstellung den chirurgischen Clip gemäß der Fig. 1 in (gespannter) Klemmposition (bei geschlossener Kopplungsvorrichtung und damit in geschlossener Konstruktionslage des Clip) und
- Fig. 3: zeigt den chirurgischen Clip gemäß der Fig. 2 in (gespannter) Offenposition (bei geschlossener Kopplungsvorrichtung).

Der chirurgische Clip gemäß einem bevorzugten Ausführungsbeispiel der Erfindung hat zwei Clipbranchen 1 jeweils zumindest bestehend aus einem Klemmabschnitt 2 und einem Betätigungsabschnitt 4 sowie einer die Clipbranchen 1 verbindenden Biegefeder oder Biegefeder - (Reihen) - Anordnung 6, die sich zumindest über einen Teilbereich längs (innerhalb/zwischen) der Betätigungsabschnitte 4 in Richtung hin zum Klemmabschnitt 2 vorzugsweise C-, U- oder V-förmig einbuchtet bzw. ausbaucht.

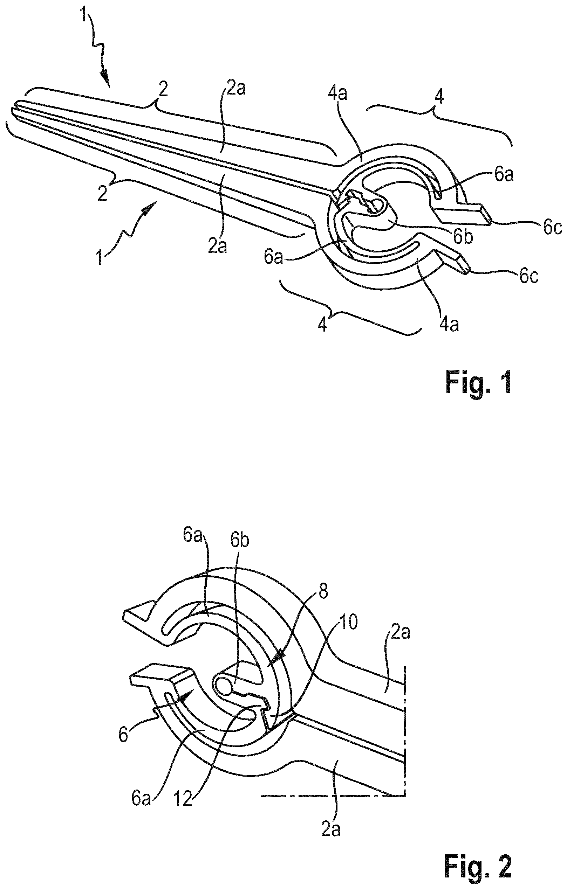

Der Klemmabschnitt 2 jeder Clipbranche 1 gemäß der Fig. 1 umfasst einen im Wesentlich geraden, langgestreckten Klemmbalken 2a, der an einer, dem anderen Klemmbalken 2a zugewandten Seite vorzugsweise mit einer Zahnung besetzt oder mit einer Riffelung versehen ist (nicht weiter dargestellt). Er kann aber auch mit einer rauen Oberfläche oder einfach nur glatt (unbearbeitete Oberfläche) ausgebildet sein. Auch können die Klemmabschnitte 2 eine völlig andere Form haben, wie beispielsweise eine maulartige Formgebung.

Die vorderen (distalen) freien Enden jedes Klemmbalkens 2a sind insbesondere im Fall der geraden Ausführung abgerundet oder mit einer Schutzkappe versehen, um Verletzungen vorzubeugen. Darüber hinaus kann jeder Klemmbalken 2a mit einer Verstärkungsleiste vorzugsweise an einer zum anderen Klemmbalken 2a abgewandten Seite versehen oder ausgebildet sein, die eine höhere Biegesteifigkeit jedes Klemmbalkens 2a zumindest in Klemm- oder Wirkrichtung gewährleistet.

Am hinteren (proximalen) Ende jedes Klemmbalkens 2a schließt sich axial unter Ausbildung eines (stumpfen) Winkels der Betätigungsabschnitt 4 in Form eines vorliegend (kreis-) bogenartig ausgebildeten Betätigungsbalkens (Jochs) 4a an, derart, dass die beiden sich gegenüberliegenden Betätigungsbalken 4a des Clip in deren Erstreckungsrichtung einen kreisförmigen Abstandsbereich fast völlig umschließen.

Im gezeigten Ausführungsbeispiel sind die Betätigungsbalken 4a im Wesentlichen langgezogen und unprofiliert ausgebildet. Sie können aber auch zumindest an ihren jeweils abgewandten (Außen-) Seiten eine an die menschliche Hand (Finger) angepasste (z.B. leicht gewellte/geschwungene) Längsform haben oder es kann eine Betätigungstaste (nicht gezeigt) auf die Betätigungsbalken 4a aufgesetzt (aufgelötet/geklebt) sein.

Erfindungsgemäß sind die Betätigungsbalken 4a biegestarr sowie stoffeinstückig mit den Klemmbalken 2a ausgeführt. D.h., die Betätigungsbalken 4a sind dafür vorgesehen, sich bei der Betätigung des chirurgischen Clips nicht (nur vernachlässigbar) elastisch/plastisch zu verformen (nicht zu verbiegen). Sie stellen somit im bevorzugten Ausführungsbeispiel keinen Teil oder Bestandteil der Biegefederanordnung 6 dar sondern dienen ausschließlich zur Kraftübertragung der Federkraft auf die Klemmabschnitte 2. Alternativ hierzu können sie aber auch über deren gesamte Länge zumindest eine gewisse Biegeelastizität besitzen (also zur Biegefederanordnung gehören) oder nur über eine Teillänge, vorzugsweise dem hinteren Balkenbereich elastisch und damit teilweise zur Biegefederanordnung gehörig, ausgebildet sein.

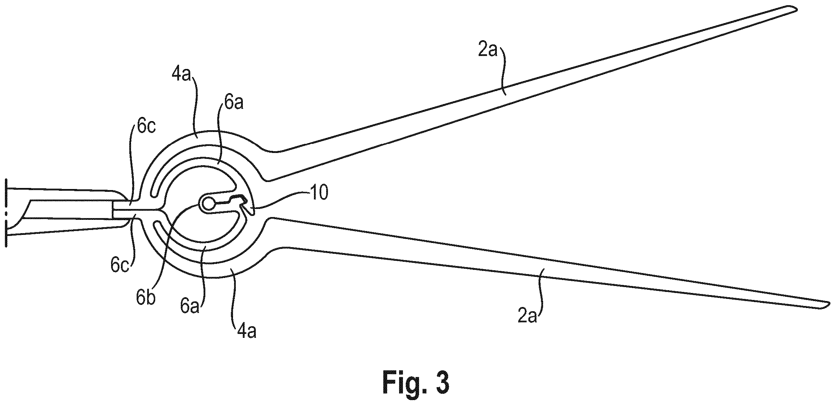

Die freien hinteren (proximalen) Enden der Betätigungsbalken 4a gehen jeweils unter Ausbildung einer Anschlagsplatte 6c in die Biegefeder bzw. einen Bestandteil (Teilbereich) der Biegefeder - (Reihen) - Anordnung 6 über. Die beiden Anschlagsplatten 6c sind dabei so zueinander ausgerichtet, dass sie bei Betätigen der Betätigungsbalken 4a aufeinander flächig zu liegen kommen und somit einen Betätigungsstopp darstellen. Gleichzeitig ragen die Anschlagsplatten 6c in einem Winkel über die Betätigungsbalken 4a in proximale Richtung vor, sodass hierdurch Angriffsabschnitte für eine externe Klemme oder Zwinge (siehe Fig. 3) geschaffen werden, über welche die Anschlagplatten 6c zusammengepresst werden können, um den Clip mechanisch in Offenposition temporär zu halten.

An dieser Stelle sei darauf hingewiesen, dass der chirurgische Clip gemäß der vorliegenden Erfindung in der Fig. 1 in Herstellungslage gezeigt ist, d.h. in einer Lage zum Zeitpunkt seiner Fertigung in ungespanntem Zustand. In diesem Zustand verlaufen die beiden Clipbranchen 1 im Wesentlichen parallel zueinander, wobei sich die Biegefeder - (Reihen) - Anordnung 6 am proximalen Ende der Betätigungsabschnitte 4 vorliegend stoffeinstückig sowie ungespannt anschließt.

Im Nachfolgenden wird die Biegefeder - (Reihen) - Anordnung 6 anhand der Fig. 1 näher beschrieben.

Die Biegefeder - (Reihen) - Anordnung 6 besteht vorliegend aus zwei äußeren Federarmen oder Federabschnitten 6a, deren jeweils proximale Enden mit den zugehörigen Betätigungsabschnitten 4 / Betätigungsbalken 4a an deren proximalen Enden verbunden sind. Dabei erstrecken sich die äußeren Federarme 6a längs der Betätigungsabschnitte 4 in Richtung distal und folgen dabei unter Beibehaltung eines im Wesentlichen gleichmäßigen Zwischenspalts der (Bogen-) Form der Betätigungsbalken 4a. Grundsätzlich besteht aber auch die Möglichkeit, dass die Form der äußeren Federarme 6a von der Form der Betätigungsbalken 4a abweicht. Vorteilhaft ist es jedoch, wenn, wie im vorliegenden Ausführungsbeispiel, die freien distalen Enden der äußeren Federarme 6a sich aufeinander zu bewegen und so den Abstand zueinander verringern. Sie zeichnen somit im gemeinsamen Grundriss in etwa eine U-, V- oder C-Form.

Die beiden äußeren Federarme 6a sind an ihren jeweiligen freien distalen Enden/Endabschnitten über ein Zwischenstück, vorzugsweise einen inneren Federabschnitt 6b (stoffeinstückig) miteinander gekoppelt. Der innere Federabschnitt 6b hat per se ebenfalls eine U-, V- oder C-Form und baucht sich in Richtung proximal aus. D.h. die beiden Federschenkel des inneren Federabschnitts 6b erstrecken sich ebenfalls in Richtung distal hin zu den Klemmabschnitten 2 und enden an der Verbindungsstelle mit den äußeren Federarmen 6a. Auf diese Weise erhält die Biegefeder - (Reihen) - Anordnung 6 gemäß dem bevorzugten Ausführungsbeispiel der Erfindung im Wesentlichen eine W - oder ω - Form mit zwei äußeren langen Federarmen 6a und zwei seriellen inneren kurzen Federschenkeln 6b. Alternativ hierzu ist es aber auch möglich, das Zwischenstück lediglich als Scharnier ohne (oder nur mit geringer) eigene Federeigenschaft auszubilden.

Darüber hinaus ist die Biegefeder - (Reihen) - Anordnung 6 mit einem Kopplungsmechanismus (Kopplungsvorrichtung) 8 zur Vorspannung der äußeren Federarme 6a und ggf. zur Veränderung der aktiven Federlänge ausgerüstet, wie nachfolgend anhand der Fig. 2 beschrieben wird.

Die Kopplungsvorrichtung 8 besteht (wird gebildet) vorliegend aus einem integral (stoffeinstückig) mit der Biegefeder - (Reihen) - Anordnung 6 ausgeformten Rastmechanismus, mittels dem das Zwischenstück (der innere Federabschnitt 6b oder Scharnier) unter elastischer Verformung der äußeren Federarme 6a quasi kurzgeschlossen werden kann.

In anderen Worten ausgedrückt hat der Rastmechanismus 8 eine hakenförmige Rastnase 10, die sich vom distalen Ende eines der äußeren Federarme 6a in Richtung hin zum distalen Ende des anderen äußeren Federarms 6a erstreckt und eine Rastleiste 12, die an dem anderen äußeren Federarm 6a ausgeformt ist und eine Art Verlängerung zu dem daran angeschlossenen Federschenkel des inneren Federabschnitts 6b (Zwischenstücks) darstellt. Die Länge (Längserstreckung) der Rastnase 10 ist so bemessen, dass für ein formschlüssiges Hintergreifen der Rastleiste 12 der innere Federabschnitt (d.h. die beiden inneren Federschenkel) elastisch (im Fall eines Scharniers plastisch) zusammengedrückt werden muss, wodurch sich die beiden äußeren Federarme 6a an deren distalen freien Enden federelastisch aufeinander zu bewegen. Der hierdurch entstehende, eingerückte Zustand der Kopplungsvorrichtung 8 ist in der Fig. 2 dargestellt.

In diesem Zustand ist nicht nur der innere Federabschnitt 6b quasi kurz geschlossen (d.h. aus dem Kraftfluss der Biegefeder - (Reihen) - Anordnung 6 genommen), um die Federcharakteristik der Biegefeder - (Reihen) - Anordnung 6 ggf. zu ändern, sondern die äußeren Federarme 6a sind nunmehr auch vorgespannt, wodurch sich die Klemmkraft auf die Klemmabschnitt in geschlossener Position (zusätzlich) erhöht.

Um den medizinische Clip zu öffnen, wie dies in der Fig. 3 gezeigt ist, werden die beiden Anschlagsplatten 6c entgegen der Federkraft bei geschlossener Kopplungsvorrichtung 8 aufeinander zu bewegt, wodurch sich die Klemmabschnitte 2a voneinander beabstanden. Um die Offenposition zu halten, kann eine zusätzliche Zwinge an die Anschlagsplatten 6c angesetzt werden, welche diese zusammen hält.

Theoretisch besteht aber auch die Möglichkeit, zunächst die Kopplungsvorrichtung 8 manuell auszurücken, indem die Rastnase 10 aus der Verrastung mit Rastleiste 12 gedrückt wird, wodurch der innere Federabschnitt 6b wieder in den Kraftfluss der Biegefeder - (Reihen) - Anordnung 6 geschaltet wird. Dadurch beabstanden sich die beiden distalen freien Enden der äußeren Federarme 6a voneinander und die Vorspannung wird freigegeben. In diesem Zustand kann nunmehr der Clip entgegen der nun nicht mehr vorgespannten, durch Aufheben des Kurzschlusses biegeweicheren Biegefeder - (Reihen) - Anordnung 6 leicht geöffnet und in der maximalen Position mittels der Klemme oder Zwinge (siehe Fig. 3) gehalten werden, die an den nunmehr aneinander liegenden Anschlagsplatten 6c angesetzt wird.

Abschließend sei erwähnt, dass der erfindungsgemäße Clip vorzugsweise als Spritzgussbauteil (einschließlich der Federanordnung) hergestellt ist. Er kann aber auch als Blechteil aus einem einzigen Material beispielsweise durch Stanzen, Drahterodieren oder Lasern insbesondere in der in Fig. 1 gezeigten Form hergestellt und vorzugsweise zumindest in den als Biegefedern vorgesehenen Bereichen gehärtet werden, um eine bestimmte Federelastizität zu erhalten. Alternativ ist es aber auch möglich, die Federanordnung zumindest bestehend aus den äußeren Federarmen 6a und dem Zwischenstück (innerer Federabschnitt 6b) oder zusammen mit den Betätigungsbalken 4a getrennt zu den Klemmabschnitten 2 aus einem Federstahl herzustellen (z.B. durch Schneiden, Stanzen oder Lasern) und dann mit den Klemmabschnitten 2 zu verbinden, beispielsweise durch Schweißen oder Hartlöten.

Die in den Fig. 1 bis 3 gezeigte Grundrissform der beiden äußeren Federarme 6a, nämlich C- oder kreisbogenförmig hat darüber hinaus den Vorteil, dass eine im Wesentlichen durchgehend konstante Biegespannung in den Federarmen 6a realisiert wird, wodurch die Materialeigenschaften bestmöglich ausgenützt werden können. Zusätzlich wird die Verschlusskraft aufgrund reproduzierbarer Fertigungsverfahren sehr konstant gehalten.

Offenbart wir zusammenfassend ein chirurgischer Clip mit zwei Clipbranchen 1 jeweils bestehend aus einem vorzugsweise gerade sich erstreckenden (balkenförmigen) Klemmabschnitt 2 und einem Betätigungsabschnitt 4 sowie mit einer Biegefederanordnung 6, über welche die beiden Clipbranchen vorzugsweise einstückig miteinander gekoppelt sind. Erfindungsgemäß ist die Biegefederanordnung 6 zumindest teilbereichsweise (mittig) so angeordnet und ausgeformt, dass sie sich zwischen den Clipbranchen 1 längs der Betätigungsabschnitte 4 in Richtung hin zu den Klemmabschnitten 2 ausbaucht. Die Biegefederanordnung 6 ist unabhängig davon durch eine Anzahl seriell gekoppelter gegenläufig sich erstreckender Einzelfederarme aufgebaut. Des Weiteren hat die Biegefederanordnung einen Kopplungsmechanismus zum wahlweisen Kurzschließen zumindest eines Abschnitts der Federanordnung zur Erreichung einer Federvorspannung in Konstruktionslage und ggf. zur Veränderung der Federcharakteristik.

Der Klemmabschnitt ist vorzugsweise definiert als jener Axialabschnitt einer Branche, der sich bei einer Bewegung des zugehörigen (daran anschließenden) Betätigungsabschnitts in Richtung hin zum gegenüberliegenden Betätigungsabschnitt vom gegenüberliegenden Klemmabschnitt entfernt. Der Dreh- oder Schwenkpunkt der beiden Branchen ist definiert durch die Federarme der Biegefederanordnung, welche die beiden Clipbranchen verbinden und vorzugsweise zusätzlich durch die Betätigungsabschnitte, die zumindest über eine Teillänge oder ganz federelastisch sind.

## Patentansprüche

1. Chirurgischer Clip mit zwei Clipbranchen (1) jeweils bestehend aus einem Klemmabschnitt (2) und einem Betätigungsabschnitt (4), sowie mit einer Biegefederanordnung (6), über welche die beiden Clipbranchen (1) miteinander gekoppelt sind, **dadurch gekennzeichnet, dass** die Biegefederanordnung (6) eine Kopplungsvorrichtung (8) hat, mittels der ein Teil der Biegefederanordnung (6) zur Vorspannung des in Herstellungslage zunächst ungespannten chirurgischen Clips aus dem relevanten Kraftfluss genommen wird, und dass der chirurgische Clip einteilig ist, wobei die Biegefederanordnung (6) aus einer Mehrzahl von seriell gekoppelten Federabschnitten (6a, 6b) aufgebaut ist, wobei die Biegefederanordnung (6), ausgehend von den Betätigungsabschnitten (4) der Clipbranchen (1), sich zumindest teilbereichsweise in Richtung zu den Klemmabschnitten (2) hin erstreckt.

2. Chirurgischer Clip nach Anspruch 1, **dadurch gekennzeichnet, dass** die Kopplungsvorrichtung (8) zwischen zumindest zwei Federarmen (6b) derart angeordnet ist, um diese in eingerücktem Zustand zumindest abschnittsweise kurz zu schließen.

3. Chirurgischer Clip nach Anspruch 1 oder 2, **dadurch gekennzeichnet, dass** die Kopplungsvorrichtung (8) längs der Biegefederanordnung (6) so positioniert und/oder dimensioniert ist, dass zu deren Einrücken ein elastisches Biegen zumindest eines der unmittelbar miteinander zu koppelnden Federarme oder Federabschnitte (6a) zur Erzeugung oder Erhöhung einer Federvorspannung notwendig ist.

4. Chirurgischer Clip nach einem der vorstehenden Ansprüche, **dadurch gekennzeichnet, dass** die Clipbranchen (1) durch Kurzschließen der Kopplungsvorrichtung (8) in Clip-Schließrichtung vorspannbar sind.

5. Chirurgischer Clip nach einem der vorstehenden Ansprüche, **dadurch gekennzeichnet, dass** sich die Biegefederanordnung (6) zumindest teilbereichsweise zwischen den Clipbranchen (1) längs der Betätigungsabschnitte (4) in Richtung hin zu den Klemmabschnitten (2) ausbaucht und dabei zumindest zwei längs der Betätigungsabschnitte (4) sich erstreckende äußere Federarme (6a) bildet.

6. Chirurgischer Clip nach Anspruch 5, **dadurch gekennzeichnet, dass** die beiden äußeren Federarme (6a) an ihren einen, proximalen Enden mit den Betätigungsabschnitten (4), vorzugsweise stoffeinstückig, verbunden sind und an ihren freien, in Richtung hin zu den Klemmabschnitten ausgerichteten, distalen Enden miteinander gekoppelt sind, derart, dass die äußeren Federarme (6a) gemeinsam im Wesentlichen einen U-, C- oder V-förmigen Grundriss bilden.

7. Chirurgischer Clip nach Anspruch 6, **dadurch gekennzeichnet, dass** die Kopplungsvorrichtung (8) bei Einrücken die beiden äußeren Federarme (6a) an deren distalen Enden federelastisch sowie unmittelbar miteinander verbindet.

8. Chirurgischer Clip nach Anspruch 6 oder 7, **dadurch gekennzeichnet, dass** die Biegefederanordnung (6) ein U-, V- oder C-förmiges Zwischenstück, vorzugsweise einen inneren Federabschnitt (6b) hat, über das die freien, distalen Enden der äußeren Federarme (6a) miteinander vorspannfrei verbunden sind und der sich in Richtung proximal ausbaucht.

9. Chirurgischer Clip nach Anspruch 8, **dadurch gekennzeichnet, dass** die Kopplungsvorrichtung (8) im Bereich der freien, distalen Enden der äußeren Federarme (6a) so angeordnet oder ausgebildet ist, dass das Zwischenstück, vorzugsweise der innere Federabschnitt (6b) mittels der Kopplungsvorrichtung (8) kurzschließbar ist.

10. Chirurgischer Clip nach einem der vorstehenden Ansprüche, **dadurch gekennzeichnet, dass** die Kopplungsvorrichtung (8) einen manuell betätigbaren Rastmechanismus für ein Ein- und Ausrücken der Kupplungsvorrichtung (8) hat.

11. Chirurgischer Clip nach Anspruch 10, **dadurch gekennzeichnet, dass** der Rastmechanismus eine hakenförmige Rastnase (10) hat, die sich stoffeinstückig vom freien distalen Ende des einen äußeren Federarms oder Federabschnitts (6a) in Richtung hin zum freien distalen Ende des anderen äußeren Federarms oder Federabschnitts (6a) erstreckt, an dem eine Hinterschneidung vorzugsweise in Form einer stoffeinstückigen Rastkante (12) ausgeformt ist, die mit der Rastnase (10) in Rasteingriff bringbar ist.

12. Chirurgischer Clip nach einem der Ansprüche 7 bis 9, **dadurch gekennzeichnet, dass** die Kopplungsvorrichtung (8) so dimensioniert ist, dass für deren Einrücken ein Zusammendrücken des hierbei kurzzuschließenden inneren Federabschnitts (6b) erforderlich ist, wodurch gleichzeitig die im Kraftfluss verbleibenden äußeren Federarme oder Federabschnitte (6a) in Clip-Schließrichtung vorgespannt werden.

13. Chirurgischer Clip nach einem der vorstehenden Ansprüche, **dadurch gekennzeichnet, dass** der Clip von der geraden Branchenbauart ist, dessen zwei sich gegenüberliegende Klemmabschnitte (2) jeweils einen gerade sich erstreckenden Klemmbalken (2a) vorzugsweise mit Zahn- oder Riffelungsbesatz aufweisen.

14. Chirurgischer Clip nach einem der vorstehenden Ansprüche, **dadurch gekennzeichnet, dass** die beiden Betätigungsabschnitte (4) jeweils einen Betätigungsbalken (4a) bilden, der über seine gesamte Länge oder Teillänge bezüglich der Federanordnung starr ist, wobei an jedem Betätigungsbalken (4a) proximal eine Anschlagsplatte (6c) vorgesehen ist, die mit Erreichen des maximalen Betätigungswegs zur Begrenzung der Biegebelastung aufeinander anschlagen.

15. Chirurgischer Clip nach einem der vorstehenden Ansprüche, **dadurch gekennzeichnet, dass** der Clip ein Blechbauteil ist, das vorzugsweise durch Stanzen oder Laserschneiden hergestellt wird.

16. Chirurgischer Clip nach einem der vorstehenden Ansprüche, **dadurch gekennzeichnet, dass** die Biegefederanordnung (6) aus einem Federstahl separat zu den Clipbranchen (1) gefertigt und mit den beiden Clipbranchen (1) stoffeinstückig vorzugsweise durch Löten oder Schweißen verbunden ist. **oder dass** der Clip einschließlich der Biegefederanordnung (6) einstückig aus einem einzigen Material gefertigt ist.

## Claims

1. A surgical clip comprising two clip branches (1) each consisting of a clamping portion (2) and an actuating portion (4) as well as comprising a flexural spring arrangement (6) by which the two clip branches (1) are coupled, **characterized in that** the flexural spring arrangement (6) includes a coupling device (8) by means of which a part of the flexural spring arrangement (6) can be removed from the relevant force flow for pre-tensioning the surgical clip which is initially non-tensioned in the manufacturing position and that the surgical clip is in one piece, wherein the flexural spring arrangement (6) is structured of a plurality of serially coupled spring portions (6a, 6b), wherein the flexural spring arrangement (6), starting from the actuating portions (4) of the clip branches (1), extends at least partially in the direction of the clamping portions (2).

2. The surgical clip according to claim 1, **characterized in that** the coupling device (8) is arranged between at least two spring arms (6b) so as to short-circuit the latter in the engaged state at least in portions.

3. The surgical clip according to claim 1 or 2, **characterized in that** the coupling device (8) is positioned and/or dimensioned along the flexural spring arrangement (6) so that for engaging the latter elastic bending of at least one of the spring arms or spring portions (6a) directly to be coupled to each other is required for generating or increasing a spring pre-tensioning.

4. The surgical clip according to any one of the preceding claims, **characterized in that** the clip branches (1) can be pre-tensioned by short-circuiting the coupling device (8) in closing direction of the clip.

5. The surgical clip according to any one of the preceding claims, **characterized in that** the flexural spring arrangement (6) bulges at least in sections between the clip branches (1) along the actuating portions (4) toward the clamping portions (2) and forms at least two outer spring arms (6a) extending along the actuating portions (4).

6. The surgical clip according to claim 5, **characterized in that** the two outer spring arms (6a) are connected to the actuating portions (4) preferably in one material piece at their one respective proximal ends and are coupled to each other at their free distal ends orientated toward the clamping portions so that the outer spring arms (6a) jointly form substantially a U, C or V shaped layout.

7. The surgical clip according to claim 6, **characterized in that** upon engagement the coupling device (8) interconnects the two outer spring arms (6a) at the distal ends thereof in a spring-elastic and direct manner.

8. The surgical clip according to claim 6 or 7, **characterized in that** the flexural spring arrangement (6) includes a U, V or C shaped spacer, preferably an inner spring portion (6b) by which the free distal ends of the outer spring arms (6a) are interconnected in an non-tensioned manner and which bulges in the proximal direction.

9. The surgical clip according to claim 8, **characterized in that** the coupling device (8) is arranged or configured in the area of the free distal ends of the outer spring arms (6a) so that the spacer, preferably the inner spring portion (6b), can be short-circuited by means of the coupling device (8).

10. The surgical clip according to any one of the preceding claims, **characterized in that** the coupling device (8) includes a manually operable detent mechanism for engaging and disengaging the coupling device (8).

11. The surgical clip according to claim 10, **characterized in that** the detent mechanism includes a hook-like lug (10) extending in one material piece from the free distal end of the one outer spring arm or spring portion (6a) toward the free distal end of the other outer spring arm or spring portion (6a) on which an undercut is preferably configured in the form of a one-material detent edge (12) which can be engaged in the lug (10).

12. The surgical clip according to any one of the claims 7 to 9, **characterized in that** the coupling device (8) is dimensioned so that for engagement thereof the inner spring portion to be accordingly short-circuited needs to be compressed, whereby simultaneously the outer spring arms or spring portions (6a) retained in the flow of force are pre-tensioned in the closing direction of the clip.

13. The surgical clip according to any one of the preceding claims, **characterized in that** the clip is of the straight branch design the two opposing clamping portions (2) of which have a straightly extending clamping bar (2a) preferably including a toothed or corrugated trimming.

14. The surgical clip according to any one of the preceding claims, **characterized in that** each of the two actuating portions (4) constitutes an actuating bar (4a) which is rigid over its entire length or partial length relative to the spring arrangement, wherein on each actuating bar (4a) proximally a stop plate (6c) is provided which abut against each other upon reaching the maximum actuating path for delimiting the flexural load.

15. The surgical clip according to any one of the preceding claims, **characterized in that** the clip is a sheet member preferably manufactured by punching or lasercutting.

16. The surgical clip according to any one of the preceding claims, **characterized in that** the flexural spring arrangement (6) is fabricated of a spring steel separately from the clip branches (1) and is connected in one material piece to the two clip branches (1) preferably by soldering or welding **or that** the clip including the flexural spring arrangement (6) is integrally made of one single material.

## Revendications

1. Pince chirurgicale avec deux branches de pince (1) respectivement constituées d'une section de serrage (2) et d'une section d'actionnement (4), ainsi qu'avec un ensemble de ressorts de flexion (6), par l'intermédiaire desquels les deux branches de pince (1) sont accouplées l'une à l'autre, **caractérisée en ce que** l'ensemble de ressorts de flexion (6) possède un dispositif d'accouplement (8), au moyen duquel une partie de l'ensemble de ressorts de flexion (6) est retirée du flux de forces important pour la précontrainte de la pince chirurgicale tout d'abord non tendue dans la position de fabrication, et que la pince chirurgicale est d'une seule pièce, dans laquelle l'ensemble de ressorts de flexion (6) est composé d'une pluralité de sections de ressort (6a, 6b) accouplées en série, dans laquelle l'ensemble de ressorts de flexion (6), à partir des sections d'actionnement (4) des branches de pince (1), s'étend au moins en partie en direction des sections de serrage (2).

2. Pince chirurgicale selon la revendication 1, **caractérisée en ce que** le dispositif d'accouplement (8) est disposé entre au moins deux bras de ressort (6b) de manière à court-circuiter celui-ci au moins sur certaines sections dans l'état engagé.

3. Pince chirurgicale selon la revendication 1 ou 2, **caractérisée en ce que** le dispositif d'accouplement (8) est positionné le long de l'ensemble de ressorts de flexion (6) et/ou dimensionné de sorte que pour l'engagement de celui-ci une flexion élastique d'au moins un(e) des bras de ressort ou sections de ressort (6a) destiné(e)s à être accouplé(e)s directement l'un(e) à l'autre est nécessaire pour la production ou l'augmentation d'une précontrainte de ressort.

4. Pince chirurgicale selon l'une quelconque des revendications précédentes, **caractérisée en ce que** les branches de pince (1) peuvent être précontraintes dans la direction de fermeture des pinces par court-circuitage du dispositif d'accouplement (8).

5. Pince chirurgicale selon l'une quelconque des revendications précédentes, **caractérisée en ce que** l'ensemble de ressorts de flexion (6) se bombe au moins en partie entre les branches de pince (1) le long des sections d'actionnement (4) en direction des sections de serrage (2) et ce faisant forme au moins deux bras de ressort extérieurs (6a) s'étendant le long des sections d'actionnement (4).

6. Pince chirurgicale selon la revendication 5, **caractérisée en ce que** les deux bras de ressort extérieurs (6a) sont reliés au niveau de l'une de leurs extrémités proximales aux sections d'actionnement (4), de préférence sans discontinuité de matière, et sont accouplés l'un à l'autre au niveau de leurs extrémités distales libres, orientées en direction des sections de serrage, de telle sorte que les bras de ressort extérieurs (6a) forment conjointement sensiblement un tracé en forme de U, de C ou de V.

7. Pince chirurgicale selon la revendication 6, **caractérisée en ce que** le dispositif d'accouplement (8) lors de l'engagement relie les deux bras de ressort extérieurs (6a) l'un à l'autre au niveau de leurs extrémités distales de manière élastique ainsi que de façon directe.

8. Pince chirurgicale selon la revendication 6 ou 7, **caractérisée en ce que** l'ensemble de ressorts de flexion (6) possède une pièce intermédiaire en forme de U, de V ou de C, de préférence une section de ressort intérieure (6b), par l'intermédiaire de laquelle les extrémités distales libres des bras de ressort extérieurs (6a) sont reliées l'une à l'autre sans précontrainte et qui se bombe dans la direction proximale.

9. Pince chirurgicale selon la revendication 8, **caractérisée en ce que** le dispositif d'accouplement (8) est disposé ou réalisé dans la zone des extrémités distales libres des bras de ressort extérieurs (6a), de sorte que la pièce intermédiaire, de préférence la section de ressort intérieure (6b), peut être court-circuitée au moyen du dispositif d'accouplement (8).

10. Pince chirurgicale selon l'une quelconque des revendications précédentes, **caractérisée en ce que** le dispositif d'accouplement (8) possède un mécanisme d'encliquetage pouvant être actionné manuellement pour un engagement et désengagement du dispositif d'accouplement (8).

11. Pince chirurgicale selon la revendication 10, **caractérisée en ce que** le mécanisme d'encliquetage possède un bec d'encliquetage (10) en forme de crochet, qui s'étend sans discontinuité de matière à partir de l'extrémité distale libre d'un(e) des bras de ressort ou parties de ressort extérieur(e)s (6a) en direction de l'extrémité distale libre de l'autre bras de ressort ou partie de ressort extérieur(e) (6a), sur laquelle est formé un dégagement de préférence sous forme d'un bord d'encliquetage (12) sans discontinuité de matière, qui peut être amené en prise d'encliquetage avec le bec d'encliquetage (10).

12. Pince chirurgicale selon l'une quelconque des revendications 7 à 9, **caractérisée en ce que** le dispositif d'accouplement (8) est dimensionné de sorte que pour l'engagement de celui-ci une compression de la section de ressort intérieure (6b) effectuant un court-circuitage est nécessaire, ce qui a pour effet que les bras de ressort ou sections de ressort extérieur(e)s (6a) restant dans le flux de force sont précontraint(e)s simultanément dans la direction de fermeture de la pince.

13. Pince chirurgicale selon l'une quelconque des revendications précédentes, **caractérisée en ce que** la pince est du type branche droite, dont deux sections de serrage (2) opposées présentent respectivement une barre de serrage (2a) s'étendant en ligne droite de préférence avec une bordure dentée ou cannelée.

14. Pince chirurgicale selon l'une quelconque des revendications précédentes, **caractérisée en ce que** les deux sections d'actionnement (4) forment respectivement une barre d'actionnement (4a), qui est rigide par rapport à l'ensemble de ressorts sur toute sa longueur ou longueur partielle, dans laquelle une plaque de butée (6c) est prévue sur chaque barre d'actionnement (4a) de manière proximale, qui sont en butée l'une sur l'autre avec l'atteinte du trajet d'actionnement maximal pour la limitation de la charge de flexion.

15. Pince chirurgicale selon l'une quelconque des revendications précédentes, **caractérisée en ce que** la pince est une pièce en tôle, qui est fabriquée de préférence par estampage ou découpage laser.

16. Pince chirurgicale selon l'une quelconque des revendications précédentes, **caractérisée en ce que** l'ensemble de ressorts de flexion (6) est fabriqué à partir d'un acier à ressorts séparément des branches de pince (1) et est relié aux deux branches de pince (1) sans discontinuité de matière de préférence par brasage ou soudage ou que la pince y compris l'ensemble de ressorts de flexion (6) est fabriquée d'une seule pièce à partir d'un seul matériau.
